# EUROPEAN PATENT APPLICATION

(11) **EP 3 628 284 A1**
(43) Date of publication of application: **01.04.2020**
(21) Application number: 18196660.7
(22) Date of filing: 25.09.2018
(51) Int. Cl.: A61F 11/14, H04R 1/10, H04R 1/34

(54) **HEARING PROTECTION DEVICE WITH SOUND REFLECTORS**

(71) Applicant: GN Hearing A/S, 2750 Ballerup (DK)
(72) Inventor: RAFT, Casper Silbo, DK-2750 Ballerup (DK)
(74) Representative: Aera A/S

(57) **Abstract**

A hearing protection device comprising a first ear protector, the first ear protector comprising at least a first sound attenuation body, a first receiver, and a first primary microphone, the first sound attenuation body comprising a cup portion providing a noise dampening space, the cup portion having a peripheral part comprising a forward facing part, a rearward facing part, an upper part and a lower part, the cup portion further comprising: an outer surface facing the ambient space; an inner cup portion accommodating the first receiver; a primary sound reception area positioned in a central area of the outer surface of the cup portion, where the central area is in an area which is between 20 and 80 % of the length between the forward facing part and the rearward facing part; and at least a first sound reflector configured to reflect and direct sound waves towards the primary sound reception area, where the first sound reflector is positioned in an area on the outer surface between the sound reception area and the peripheral part of the cup portion.

## Description

### TECHNICAL FIELD

The present disclosure relates to a hearing protection device.

### BACKGROUND

Hearing in combat and/or emergency situations, it may be desirable for a user to protect his/her hearing while enabling the user to communicate with others via radio communication. During combat situations a user may need to wear a hearing protector, to attenuate noise from gunfire, machinery and other types of constant or intermittent noise.

A drawback of using a hearing protection device may be that normal sounds, such as speech, or other environmental sounds may be attenuated, causing the user to struggle in hearing what is going on around him. One solution for this has been to provide the hearing protector with forward facing microphones that allow the user to hear sounds that are received by the microphones, that are positioned close to the face of the user. Due to this positioning the user cannot recognize the direction where the sound is coming from, i.e. whether the sound is coming from behind, from the side, above or other positions, especially where the headset is configured to pass a hear-through function of the sound through the hearing protection device.

The hearing of a normal person is highly advanced and the processing of the hearing in the brain is a highly complex process, where one of the factors that the brain takes into account is head related transfer functions of the sound, where the sound is reflected of structures of the head, such as the outer ear of the user. All of this mechanism is bypassed using conventional hear-through functions using forward facing microphones, which means that the brain of the user is not able to recognize the direction of a sound.

### SUMMARY

Thus, there is a need to improve the hear-through function of a hearing protection device.

Accordingly, a hearing protection device is provided, the hearing protection device comprising a first ear protector, the first ear protector comprising at least a first sound attenuation body, a first receiver, and a first primary microphone, the first sound attenuation body comprising a cup portion providing a noise dampening space, the cup portion having a peripheral part comprising a forward facing part, a rearward facing part, an upper part and a lower part, the cup portion further comprising: an outer surface facing the ambient space; an inner cup portion accommodating the first receiver; a primary sound reception area positioned in a central area of the outer surface of the cup portion; and at least a first sound reflector configured to reflect and direct sound waves towards the primary sound reception area, where the first sound reflector is positioned in an area on the outer surface between the sound reception area and the peripheral part of the cup portion. The central area may be in an area which is between 20 and 80 % of the length between the forward facing part and the rearward facing part

The hearing protection device is configured to protect the hearing of a user using a sound attenuation body, where the sound attenuation body provides a predefined sound attenuation for the user from sounds that occur in the surroundings of the user. Such sounds may be sounds that have such a high volume so that a single instance or a constant exposure to the sounds may become damaging to the hearing of the user. The sound attenuation body blocks or reduces the magnitude of the sound to the user. The sound attenuation body on its own is non-distinctive in what sounds are attenuated, and reduces the volume of all sounds to the user. Thus, in order to make it easier for the user to operate using the hearing protection device, the hearing protection device may be configured with a hear-through function, where the hear through function allows certain sounds to be transmitted from the outside of the hearing protection device and to the user. This may e.g. be sounds such as voices, so that the user is capable of conversing with his surroundings without having to take off the hearing protection device. Other types of sounds may e.g. be low level ambient sounds which the user would hear without the use of the hearing protection device.

It is an advantage of the present disclosure, that the hearing protection device can emulate at least part of the head related transfer functions to enable or allow the user to recognize the direction of soundwaves that come into contact or fall upon the hearing protection device. Further, the present disclosure reduces the need for highly complex processing of the microphone input signals to maintain directional cues for the user.

One issue relating to traditional hear hear-through functions or processing is that the user in most cases is not capable of identifying which direction a sound comes from, as the microphones often override the functionality of the brain, to calculate where a sound is coming from using only the ears. The hearing processing in the brain utilizes the body of the user, and especially the outer ear, and the head, to calculate/determine where sound is coming from, as the brain knows how sound reflects of the physical structures of the body, and can therefore process the signals in a certain way, to identify e.g. the direction of the sound. A microphone is often positioned on hearing protection devices in a front area of the hearing protection device, which means that it is difficult for the user to identify where the sound is coming from.

With the present hearing protection device, the outer surface of the cup portion is provided with a sound reflection structure, that allows the sound to be reflected of this structure before it is received by a microphone, in a similar manner as an outer ear of a user functions. Thus, if a sound comes from a certain source, the sound wave may arrive at the sound reception area in a direct manner, as well as a reflection of the sound reflector, meaning that a sound originating from one source is received at two different times. The brain knows how to process this sound, meaning that this allows the user unconsciously to identify the direction of the sound. This is further assisted by utilizing the hearing of the opposite ear, as this also allows the brain to process the same sound from a completely different direction. Thus, the provision of a sound reflector on the hearing protection device, may increase the complexity of the sound received by a microphone, and where this sound is transmitted onwards to a receiver, where the receiver may reproduce the sound with the complexity of how it was received. Thus, the user may receive the sound in a similar manner as he would do without the use of the hearing protection device.

The sound reception area of the hearing protection device may be seen as the area where the sound is intended to be focused, similar to a focus point of a lens. The sound reception area may be the area where a microphone is positioned, or it may be a part that forwards the sound to a microphone that is positioned elsewhere.

The forward facing part, rearward facing part, upper part and the lower part of the cup portion may be defined in view of how the cup portion is configured to the worn by the user, where the forward facing part faces forward/the front part of the head, the rearward facing part faces backward/the back of the head, the upper part faces upwards/the top of the head, and the lower part faces downwards/the body of the user. The peripheral part of the cup portion may be an annular part, the cup portion having a diameter that may be adapted for the ear of the user, so that the peripheral area at least covers and/or encircles the ear of the user. The cup portion may have an outer portion that faces the ambient space, and an inner cup portion that is configured to face the ear of the user. The inner cup portion may be provided with a sound-deadening or sound-attenuating material, such as an acoustic foam which is capable of absorbing sound waves by increasing air resistance and thereby reducing the amplitude of the soundwaves, which may assist in the attenuation of sounds from the ambient space. The receiver may be in the form of a speaker, which may be used to transmit sound to the ear of the user, where the speaker is often arranged between the sound-deadening material and the ear, allowing the sound from the receiver to reach an ear of the user.

The present hearing protection device may be a passive hearing protection device, where the ambient sounds are physically blocked and attenuated by the structure of the sound attenuation body. Alternatively, or in combination, the hearing protection device and/or a communication device connected to the hearing protection device may be provided with active noise reduction, where the microphone may be utilized to capture a signal, and electronics may create phase shifted signal cast out of a receiver and/or a speaker to reduce the amplitude of the signal received by the microphone. The electronics may be included in the hearing protection device and/or in the communication device connected to the hearing protection device.

The sound reception area is in accordance with the present hearing protection device located in a central part of the outer surface of the cup portion. The central positioning of the sound reception area allows sounds from any side to be received by the sound reception area, i.e. that the sound may be received from all directions, in the same manner as the auditory canal is positioned on the side of a human head.

The central area may be an area that is between 20-80% of the width of the cup portion from its forward facing part and the rearward facing part. The central area may be between 30-70% of the width of the cup portion, or alternatively between 40-60% of the width. The central area may have a tangential axis, where the sound reception area is located is close to being parallel to a tangential axis of the side of the head, so that the central area is somewhat parallel to the side of the head, similar to the opening of the auditory canal of a human ear.

As sound strikes the user, the size and shape of the head, ears, ear canal, density of the head, size and shape of nasal and oral cavities, all transform the sound and affect how the sound is perceived, boosting some frequencies and attenuating others, where the brain takes all of these factors into consideration. The provision of the present hearing protection device is intended to provide a more complex sound image for the brain to process, by replicating some of the structural features that would otherwise be bypassed by the provision of a hearing protection device.

The cup portion may be a cup formed shell, having at least one sound attenuating wall, where the outer side of the wall is intended to face the ambient space, while the opposing inner side of the wall is intended to face the ear of the user.

In one or more embodiments, the hearing protection device may comprise a second ear protector, the second ear protector comprising a second attenuation body, where the second ear protector is configured to be connected to the first attenuation body via a connecting element, such as a headband, helmet, etc. The second sound attenuation body may be similar to the first sound attenuation body, and have the same or similar structures, where the shape of the second sound attenuation body may have a vertically mirrored structure, so that the structures of the first attenuation body that are forward facing, are similarly forward facing on the second sound attenuation body, when they are positioned on the opposite side of the head and/or on the opposite ear of the user. The connecting element may be a head strap, a helmet, or any suitable structure that is capable of holding the first and the second sound attenuation body to the side of the head covering the ear of the user. The first and the second sound attenuation bodies may be provided with a connecting part, where the connecting part may be e.g. attached to a headwear of a user, such as a helmet, hat, or other suitable headwears, where the headwear may thereby be part of the connecting element allowing the sound attenuation bodies to the biased in a direction towards the ear of the user and hold the sound attenuation bodies in a correct position. Such connecting elements are well known to the person skilled in the art, and the person skilled in the art will recognize based on the present disclosure that any suitable structural element may be utilized for connecting the first attenuation body to the second attenuation body.

In one or more embodiments, the first sound reflector may be positioned between the rearward facing part of the peripheral part and the primary sound reception area. The sound reflector may be positioned in an area to emulate the function of e.g. the Antihelix of an ear of a user, where the first sound reflector may reflect sounds that originate in a direction from the forwards facing part and reflect the sound towards the sound reception area.

In one or more embodiments, the cup portion may further comprise a second sound reflector, where the second sound reflector is positioned between the upper part of the peripheral part and the primary sound reception area. The sound reflector may be positioned in an area to emulate the function of the Helix, spine of Helix or the Triangular Fossa of an ear of a user, where the first sound reflector may reflect sounds that originate in a direction from the upper part and reflect the sound towards the sound reception area.

In one or more embodiments, the cup portion may further comprise a third sound reflector, where the third sound reflector is positioned between a lower part of the peripheral part and the primary sound reception area. The sound reflector may be positioned in an area to emulate the function of the Antitragus of an ear of a user, where the first sound reflector may reflect sounds that originate in a direction from the lower part and reflect the sound towards the sound reception area.

In one or more embodiments, the cup portion further may comprise a fourth sound reflector, where the fourth sound reflector is positioned between the forward facing part of the peripheral part and the primary sound reception area. The fourth sound reflector may be positioned in an area to emulate the function of the tragus of an ear of a user, where the fourth sound reflector may reflect sounds that originate in a direction from the rearward facing part and reflect the sound towards the sound reception area. The fourth sound reflector may also be utilized having a function of a funnel, so that sound received from e.g. another sound reflector may be directed towards the sound reception area.

The provision of a sound reflector allows the sound coming from an emanating source to be received in at least two separate points in time, as the reflected sound wave has to travel a longer distance than a sound that is received directly at the sound reception area. Thus, the provision of at least one sound reflector allows the complexity of the sound to be increased, where the increased complexity of the sound may be processed by the brain of the user, as this complex sound is closer to the type of sound which the user is already familiar with, when the user is not using a hearing protection device. Thereby, the need for complex processing of microphone input signals to emulate the head related transfer functions is reduced.

Any one or more of the sound reflectors that are positioned on the outer cup portion may also function as a blocking structure, where the sound coming from a direction that has to pass the sound reflector may be attenuated by the sound reflector, and thereby reduce in amplitude before it reaches the sound reception area. This is intended to emulate the physical structures of the ear, where the structures both operate as a sound reflection structure as well as an attenuation element. This may increase the complexity of the sound received at the sound reception area, allowing the processing of the brain to be similar to what is occurring when the user is not using a hearing protection device, and thereby the physical structures of the hearing protection device are intended to emulate the physical structures of the ear, to increase the sound complexity, not only as a sound reflector, but also as an attenuation element.

In one or more embodiments, the cup portion may comprise a channel extending from the sound reception area in a direction towards the front facing part of the peripheral edge, where the at least first sound reflector optionally abuts the channel. The channel extending from the sound reception area and forwards may emulate the Concha of the outer human ear, so that the channel may operate as a structure that directs the sounds towards the sound reception area. This may both be any sounds that intersect the channel directly from the source of the sound or sounds that intersect the channel indirectly from the source of the sound, such as sounds that reflect off sound reflectors or of the side of the head, such as sounds that are transformed by the head or the body of the user.

In one or more embodiments, the sound reception area may comprise a microphone opening for directing incoming sound to the first primary microphone. The microphone opening may allow the microphone to be positioned in an area that is away from the outer surface of the cup portion. The microphone opening may collect sounds that are directed towards the sound reception area and/or the opening and lead the sounds towards the microphone via the microphone opening. The opening may allow the microphone to be positioned away from the sound reception area, and where the microphone may e.g. be positioned on an inner side of a wall of the cup portion, while the sound reception area is on the opposing outer side of the wall.

The microphone opening may be provided with a membrane or filter element, where the membrane/filter element may provide e.g. water and dust protection to the microphone and/or any elements of the inner cup portion while still allowing sound to pass the membrane/filter element without attenuating the sound, or at least without attenuating the sound in a significant way.

In one or more embodiments, the sound reception area may be offset from the outer surface of the cup portion in a radial direction. The radial direction may be a direction that is substantially normal to the side of the head, and/or a plane defined by the peripheral part of the cup portion. By offsetting the sound reception area from the outer surface of the cup portion it is possible to ensure that sounds that emanate from a plane that is tangential to the outer surface of the cup portion cannot reach the sound reception area directly but can only be received by reflecting the sounds by physical structures into the sound reception area. Thus, the sounds that are directed to the sound reception area have a vector that at least partially has a direction that is parallel to the radial direction, where the sounds still may have vectors in other directions in a three dimensional space, the other directions being different from the radial direction. Thus, the sound reception area may be moved from the outer surface of the cup portion, and thereby increasing the complexity of the sound when the sound interacts with the sound reception area.

In one or more embodiments, the sound reception area may comprise an opening in the outer surface of the cup portion. The opening in the outer surface of the cup portion may allow the sound reception area to be moved from the outer surface of the cup portion and in a radial direction towards the side of the head of the user when the cup portion is in use, and allowing the sound reception area to be counter-sunk, e.g. where a microphone may be positioned inside the opening. By providing an opening and positioning the sound reception area inside the opening, a wind protector may be positioned between the outer surface and the sound reception area, which means that the sound reception area may be protected from noise that would distort the sound, such as wind.

In one or more embodiments, the sound reception area may comprise a canal extending from the outer surface of the cup portion and extending inwards towards the inner portion of the cup portion. The canal may be utilized to transfer the sound from one position to another, similar to an auditory canal of the human ear. The canal may e.g. transfer the sound from one area to another area where the sound is received by a microphone/microphone opening. The canal may either transfer the sound identically from one area to the other or may also be utilized to increase the complexity of the sound, similar to the ear canal of the user. The canal may be utilized to force the sound to bounce off structures inside the canal to e.g. to linearize the sound waves. I.e. that the direction of the sound waves is substantially parallel when the sound is received by the microphone.

In one or more embodiments, the cup portion may comprise a primary canal having a first end at and extending from the outer surface of the cup portion towards a second end. The canal may direct the sound from the outside of the outer surface and towards the second end of the canal.

In one or more embodiments, the second end of the primary canal comprises the sound reception area. Thus, the sound reception area is similar to the Tympanic membrane of the ear, where sound is received at the end of the canal and has travelled through the canal before it is received at the end of the canal. The sound reception area may comprise a microphone at the second end or may comprise a microphone opening at the end of the canal, where a microphone is positioned on the opposite side of the microphone opening.

In one or more embodiments, the sound receiving area may be configured to be positioned substantially centered relative to the ear canal of the user, when the hearing protection device is in use. By positioning the sound receiving area substantially centered relative to the ear canal of the user, the hearing protection device can utilize other head related transfer functions of the sounds, even though the outer ear is covered by the hearing protection device. The hearing processing of the brain is configured to a spatial recognition of the head as well as the ears, and by positioning the sound receiving area in the same central area as the ear canal, it may be possible to simplify the processing of the sound by the brain by introducing the sounds in a similar position to what the body is used to. The meaning of the term "substantially centered relative to the ear canal of the user" means that a normal emanating from the ear canal of the user will intersect the sound reception area. The normal is substantially orthogonal to the side of the head and is an axis that extends outwards from the side of the head of the user. Thus, the imaginary axis will intersect both the ear canal of the user and the sound reception area.

In one or more embodiments, the first ear protector may be an over-the-ear configuration. An over-the-ear configuration is where the sound attenuation body covers the entire ear, and the peripheral part of the cup portion has a diameter that is larger than the ear of the user, in any direction. An over-the-ear hearing protector may be provided in different sizes, to accommodate different sizes of ear of a user. This essentially means that the hearing protector can be positioned over the ear of a user, without coming into contact with the ear.

In one or more embodiments, the peripheral edge of the cup portion may be provided with a cushion providing a seal between the side of the head and the cup portion. The cushion may be made of or comprise a foam material, and has an outer surface that can provide a comfortable fit to the side of the head, as well as a sound attenuating seal. The cushion may be adapted to form to the surface area of the side of the head, and where the foam is both flexible and pliable to adapt to a curved surface area.

In one or more embodiments, the hearing protection device may comprise a secondary microphone device wired to a secondary terminal of the first connector, the secondary microphone device configured for picking up own voice of a user of the hearing protection device. The secondary microphone may be a boom microphone, which is attached to the hearing protector and extends along a boom and can be configured to be positioned in front of or near the mouth of the user for picking up and transmitting the user's voice to e.g. radio communication.

### BRIEF DESCRIPTION OF THE DRAWINGS

The above and other features and advantages of the present invention will become readily apparent to those skilled in the art by the following detailed description of exemplary embodiments thereof with reference to the attached drawings, in which:
Fig. 1 schematically illustrates an exemplary hearing protection system,
Fig. 2 is an end view of an exemplary first connector of the hearing protection device
Fig. 3 is an end view of an exemplary second connector of the communication device,
Fig. 4 is a schematic block diagram of a processor of an exemplary communication device,
Fig. 5 schematically illustrates an exemplary hearing protection device,
Fig. 6 is a perspective view of a cup portion,
Fig. 7 is a perspective view of a cup portion, and
Fig. 8 is a side view of a cup portion.

### DETAILED DESCRIPTION

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

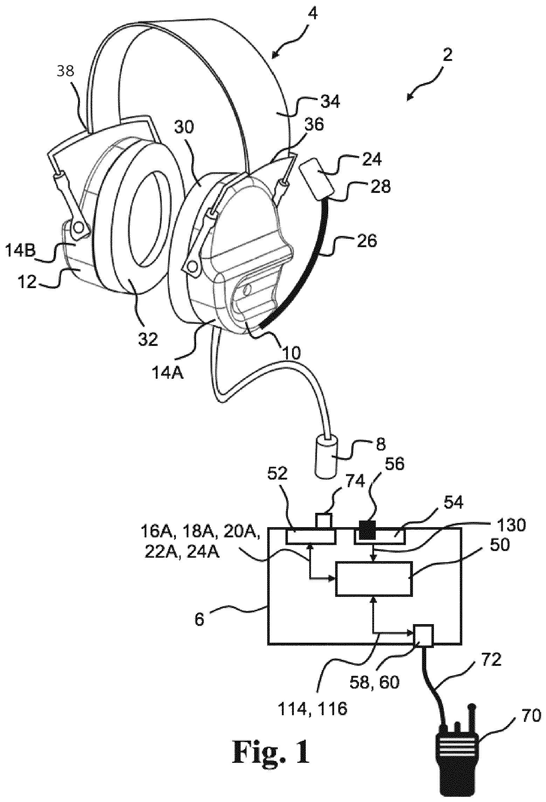

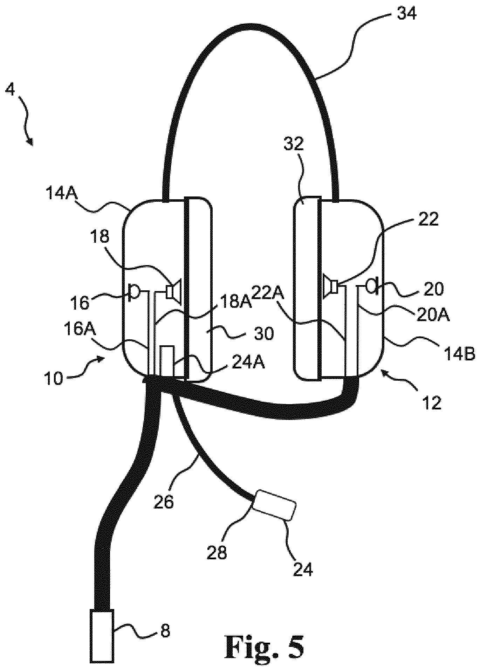

Fig. 1 shows an exemplary hearing protection system. The hearing protection system 2 comprises a hearing protection device 4 and a communication device 6. The hearing protection device 4, see also Fig. 5, comprises a first connector 8; a first ear protector 10; and a second ear protector 12. The first ear protector 10 comprises a first sound attenuation body 14A, a first primary microphone 16, and a first receiver 18, wherein the first primary microphone 16 and the first receiver 18 are electrically connected to a first primary terminal and a first receiver terminal of the first connector 8, respectively, i.e. the first primary microphone 16 is electrically connected to a first primary terminal of the first connector 8 and the first receiver 18 is electrically connected to a first receiver terminal of the first connector 8. The first sound attenuation body 14A is configured to cover an outer ear of a user.

The second ear protector 12 comprises a second sound attenuation body 14B, a second primary microphone 20, and a second receiver 22, wherein the second primary microphone 20 and the second receiver 22 are electrically connected to a second primary terminal and a second receiver terminal of the first connector 8, respectively, i.e. the second primary microphone 20 is electrically connected to a second primary terminal of the first connector 8 and the second receiver 22 is electrically connected to a second receiver terminal of the first connector 8. The second sound attenuation body 14B is configured to cover an outer ear of a user.

The hearing protection device 4 comprises a secondary microphone device 24 electrically connected to a secondary terminal of the first connector, the secondary microphone device configured for picking up own voice of a user of the hearing protection device. The hearing protection device 4 comprises a microphone boom 26 with a distal end 28, and the secondary microphone device 24 is arranged at the distal end 28 of the microphone boom 26.

In hearing protection device 4, the first ear protector 10 comprises a first cushion 30 configured to encircle an outer ear of the user, and the second ear protector 12 comprises a second cushion 32 configured to encircle an outer ear of the user. The hearing protection device 4 comprising a headband 34, wherein the first ear protector 10 and the second ear protector 12 are connected to respective first end 36 and second end 38 of the headband 34.

The communication device 6 comprises a processor 50 and an interface, the interface comprising a hearing protection device interface 52 for connection to hearing protection device 4; a user interface 54 including one or more buttons, such as a first button 56; and a radio interface 58 including a third connector 60 for connecting to and communicating with a radio unit 70 or communication system/intercom system via cable 72. The processor 50 is wired to respective terminals of the hearing protection device interface 52; the user interface 54; and the radio interface 58.

The third connector 60 comprising a power terminal (not shown) for provision of power to the communication device 6 from a power unit, e.g. a battery (not shown), of the radio unit 70 connected to the third connector 60. The hearing protection device interface 52 includes a second connector 74 comprising terminals configured for mating with respective terminals of the first connector 8 of the hearing protection device 4. When the hearing protection device 4 is connected to the communication device 6, the processor 50 receives, via the second connector 74, a first primary microphone input signal 16A from the first primary microphone 16 of hearing protection device 4 and a second primary microphone input signal 20A from the second primary microphone 20 of hearing protection device 4. Further, the processor is configured to output a first output signal 18A for the first receiver 18 of hearing protection device 4, and to output a second output signal 22A for the second receiver 22 of hearing protection device 4. Further, when the hearing protection device 4 is connected to the communication device 6, the processor 50 optionally receives, via the second connector 74, a secondary microphone input signal 24A from the secondary microphone device of hearing protection device 4.

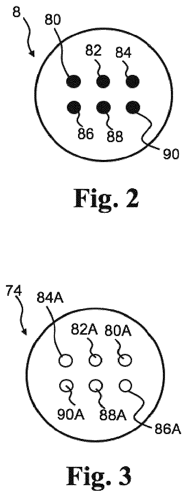

Fig. 2 is an end view of an exemplary first connector 8 of the hearing protection device 4. The first connector 8 comprises a first primary terminal 80, a first receiver terminal 82, a second primary terminal 84, and a second receiver terminal 86.

The first connector 8 may comprise a secondary terminal 88. The secondary terminal 88 is electrically connected to the secondary microphone device 24 configured for picking up own voice of a user of the hearing protection device. The first connector 8 comprises a reference terminal 90 also denoted a ground terminal.

Fig. 3 is an end view of an exemplary second connector 74 of the communication device 6. The second connector 74 comprises a first primary terminal 80A, a first receiver terminal 82A, a second primary terminal 84A, and a second receiver terminal 86A. The terminals 80A, 82A, 84A, 86A are configured for mating with respective terminals 80, 82, 84, 86 of the first connector 8 of the hearing protection device 4.

The second connector 74 may comprise a secondary terminal 88A. The secondary terminal 88A is electrically connected to the processor 50 for feeding secondary microphone input signal 24A from secondary microphone device 24 to the processor 50. The second connector 74 comprises a reference terminal 90A also denoted a ground terminal.

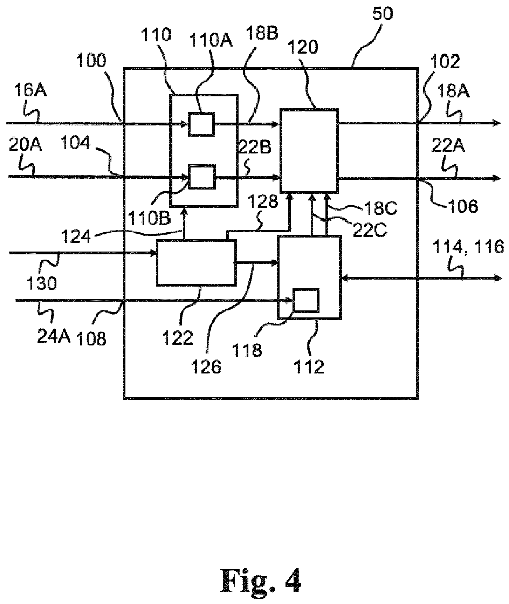

Fig. 4 shows a schematic block diagram of a processor of an exemplary communication device 6. The processor 50 has a first primary input 100 electrically connected to first primary terminal 80A of second connector 74 and configured to receive first primary microphone input signal 16A from the first primary microphone 16 of hearing protection device 4. The processor 50 has a first receiver output 102 electrically connected to first receiver terminal 82A of second connector 74 and configured to output first output signal 18A to the first receiver 18 of hearing protection device 4. The processor 50 has a second primary input 104 electrically connected to second primary terminal 84A of second connector 74 and configured to receive second primary microphone input signal 20A from the second primary microphone 20 of hearing protection device 4. The processor 50 has a second receiver output 106 electrically connected to second receiver terminal 86A of second connector 74 and configured to output second output signal 22A to the second receiver 22 of hearing protection device 4. The processor 50 has a secondary input 108 electrically connected to secondary terminal 88A of second connector 74 and configured to receive secondary microphone input signal 24A from the secondary microphone device 24 of hearing protection device 4.

The processor 50 is configured to process the first primary microphone input signal 16A for provision of a first output signal 18A for the first receiver based on the first primary microphone input signal 16A; and output the first output signal 18A to the first receiver terminal 82A of the second connector.

The processor 50 comprises hear-through processing module 110 for performing hear-through processing of the first primary microphone input signal 16A and the second primary microphone input signal 20A. The hear-through processing module 110 comprises first noise reduction module 110A for performing noise reduction, such as impulse noise reduction, on the first primary microphone input signal 16A for provision of a first primary output signal 18B forming at least part of first output signal 18A for the first receiver 18 based on the first primary microphone input signal 16A. The hear-through processing module 110 comprises second noise reduction module 110B for performing noise reduction, such as impulse noise reduction, on the second primary microphone input signal 20A for provision of second primary output signal 22B forming at least part of second output signal 22A for the second receiver 22 based on the second primary microphone input signal 20A.

The processor 50 comprises communication module 112 for provision of first secondary output signal 18C forming at least part of first output signal 18A for the first receiver 18 based on an input audio signal 114 received from the radio unit 70 via third connector 60 of radio interface 58. Further, the communication module 112 is configured for provision of second secondary output signal 22C forming at least part of second output signal 22A for the second receiver 22 based on the input audio signal 114. The communication module 112 is optionally configured to provide an output audio signal 116 for the radio unit 70 via third connector 60 of radio interface 58 based on the secondary microphone input signal 24A. The secondary microphone input signal 24A may be fed directly to the radio interface as output audio signal 116 or optionally processed in voice processing module 118. The voice processing module is optionally configured to filter and/or amplify the secondary microphone input signal 24A for provision of the output audio signal. In one or more exemplary processors, the input audio signal 114 from the radio interface is fed directly to mixer.

The processor 50 comprises mixing module 120 for provision of the first output signal 18A based on the first primary output signal 18B and the first secondary output signal 18C, e.g. by addition and/or a linear combination of the first primary output signal 18B and the first secondary output signal 18C. Further, the mixing module 114 is optionally configured for provision of the second output signal 22A based on the second primary output signal 22B and the second secondary output signal 22C, e.g. by addition and/or a linear combination of the second primary output signal 22B and the second secondary output signal 22C.

The processor 50 optionally comprises controller 122 configured to control one or more of hear-through processing module 110 (via control signal 124), communication module 112 (via control signal 126) and mixing module 120 (via control signal 128), e.g. based on user input signal 130 from the user interface 54. For example, the communication module may be controlled to only feed the secondary microphone input signal 24A as the output audio signal 116 if the user input signal 130 is indicative of a user activating the first button 56 (push-to-talk function). The user input signal from the user interface may be fed to the third connector to enable control of the radio unit 70. In one or more exemplary communication devices, the control signal 124 controls, e.g. sets and/or adjusts, one or more noise reduction parameters, such as one or more filter coefficients, of noise reduction modules 110A, 110B. In one or more exemplary communication devices, the control signal 126 controls, e.g. sets and/or adjusts, one or more voice processing parameters, such as one or more filter coefficients and/or one or more gains of voice processing module 118. In one or more exemplary communication devices, the control signal 128 controls, e.g. sets and/or adjusts, one or more mixing parameters, such as one or more gain coefficients and/or weights, of mixing module 120.

The processor 50 is powered from the radio unit 70 or communication system/intercom system via a power terminal of the third connector 60 comprising a power terminal for provision of power to the communication device from a radio unit connected to the third connector.

Accordingly, the processor 50 is configured to receive a first primary microphone input signal 16A from the first primary microphone 16; process, with the hear-through processing module 110 and mixing module 120, the first primary microphone input signal 16A for provision of a first output signal 18A for the first receiver based on the first primary microphone input signal 16A; and output the first output signal 18A to a first receiver terminal of the second connector.

Fig. 5 schematically illustrates hearing protection device 4 and components thereof. A cable with conductors connects terminals of first connector 8 with respective microphones 16, 20, 24 and receivers 18, 22. In one or more exemplary hearing protection devices, the first connector is arranged near or in an ear protector, and the first connector and second connector are connected via a cable with connectors for mating with the first and second connector, respectively.

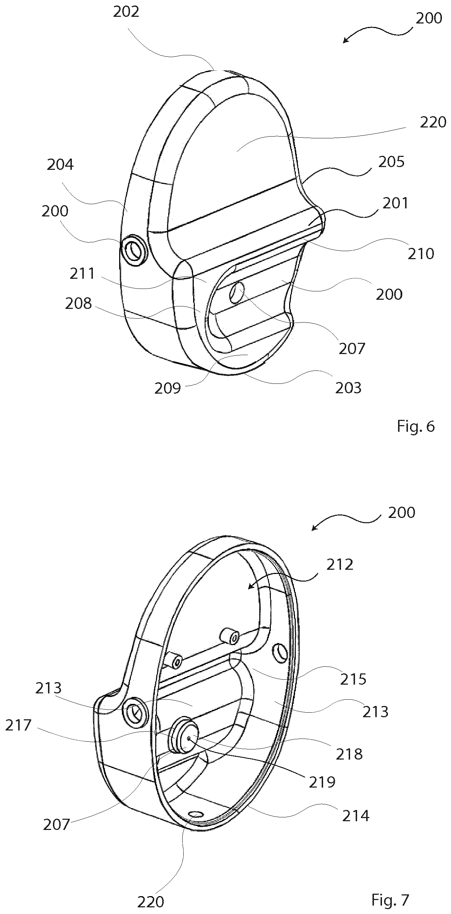

Fig. 6 shows a cup portion 200 of an exemplary first sound attenuation body 14A seen in a side perspective view of the outer surface 201 of the cup portion 200. The cup portion comprises an upper part 202, a lower part 203, a rearward facing part 204 and a forward facing part 205. The cup portion has connecting parts 206 respectively positioned on the rearward facing part 204 and the forward facing part 205 (not shown) for e.g. connecting a connecting element, such as a headband to the cup portion. The cup portion 200 comprises a microphone port 207, which is positioned in a central location between the forward facing part 205 and the rearward facing part 204. The microphone port 207 may be part of the sound receiving area, where the microphone (not shown) may be positioned at the bottom of the microphone port 207.

The microphone port 207 is arranged on a surface area that configured to be substantially parallel to the side of the head of the user, so that the microphone port points outwards in a radial direction away from the side of the head during use.

The cup portion 200 may be provided with a first sound reflector 208, a second sound reflector 209 and a third sound reflector 210, which are positioned above, behind and below the microphone port 207, in order to surround the microphone port from three sides. The sound reflectors are adapted to reflect soundwaves that come from a front facing direction, an upwards direction and a downwards direction, relative to the cup portion, towards the microphone port 207. The ear protector comprises a microphone (also denoted first primary microphone and/or second primary microphone) arranged at the microphone port 207, e.g. on the inside or on the outside of the cup portion. The microphone is adapted to receive any sounds that enter the microphone port and transforms the sound waves into electrical signals. The first 208, second 209 and third 210 sound reflectors may be connected to each other, as is shown in the present example, creating a single structure that surrounds the microphone port 207 from at least three sides. It is obvious to the person skilled in the art, that the reflector structure 211 shown in this example is only exemplary and can be adjusted and amended in various ways to obtain a similar structure, as long as it is configured to reflect sound waves towards the microphone port 207.

The reflection structure 208, 209, 210 may be seen as structural elements that are adapted to replicate parts of the physical structures of the human ear of a user. By providing the reflecting structures it is possible to get a complex sound image, where the same sound can reach the microphone port 207 at different times, allowing the brain to distinguish between the sounds, and their time difference, to identify in a better way where the sounds come from. Thus, the reflector structure 211 is intended to replicate at least parts of the sound reflecting properties of the human ear.

Fig. 7 shows the inner part 212 of the cup portion 200 seen in a perspective side view. The cup portion may be seen as being a shell 213, which has an annular peripheral part 214 which surrounds the inner volume 215 of the shell. The inner surface 216 of the cup portion 200 comprises a microphone port 207, where the microphone port comprises a side wall 217 and a bottom wall 218, where the bottom wall 218 comprises a microphone opening 219, allowing sound to be transferred from the opposite side and inwards towards the microphone (not shown) which may be attached to the bottom wall 218 to allow the microphone to receive the sounds that are received through the microphone opening 219. The lower part 203 may further be provided with a cable opening 220, which allows a cable communication cable (not shown) to be inserted into the inner volume 215 of the shell, to provide electrical communication with the microphone (not shown) and a receiver and/or a speaker (not shown). The microphone opening 219 in this example, may be seen as the primary sound reception area, where the side walls 217 and the bottom wall 218 may be seen as a canal (auditory canal) adapted to transfer the sound from the outer surface of the cup portion towards the primary sound reception area.

The microphone opening is offset from the outer surface of the cup portion (shown in Fig. 6) which means that most sounds have to be reflected of different structures before the sound waves enter the microphone opening. This means that the sound received in the microphone opening may be a sound originating from a single source, but enters the microphone opening at different time intervals, thereby creating a complex sound that is received by the microphone. This complex sound is then received by the microphone, optionally processed with hear-through processing and/or noise-cancellation, and transferred to the receiver (speaker) to output sound to the ear of the user, when the user is wearing the hearing protection device.

The inner volume 215 of the shell/cup portion may be filled or at least partly filled with a sound deadening material (not shown), in order to attenuate any sound waves that may travel through the outer wall of the shell during use, and attenuate the sound waves on their way towards the ear of the user. Furthermore, the inner volume 215 may be provided with a speaker/receiver (not shown), where the speaker is configured to output sounds based on an output signal from a sound processing element (not shown) or communication device to the ear of the user, while the cup portion 200 is worn to cover the outer ear of a user.

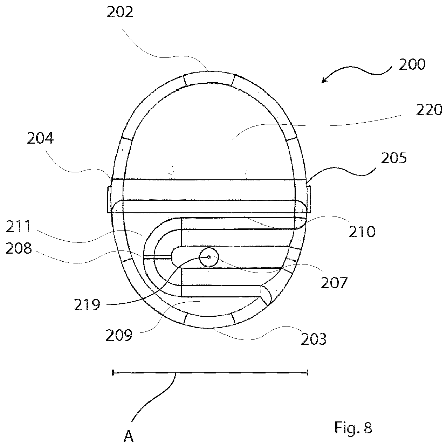

Fig. 8 shows a side view of the cup portion 200, having same reference numbers as the cup portion seen in Fig. 6 and 7, where the parts having where the axis A represents the distance from the forwards facing part 205 to the rearward facing part 204. The sound reception area (microphone port 207) can be seen as being positioned substantially equidistantly from the forwards facing part 205 and the rearward facing part 204, i.e. at the centre of the axis A. In accordance with this disclosure, the positioning of the sound reception area may be between 20% and 80% of the distance from the rearward facing part and the forward facing part, which effectively means that the sound reception area is not on the forward facing parts and the rearward facing parts, and is directed outwards from the side of the head. Optionally, the sound reception area is aligned with or positioned directly in extension to the ear canal of the user, when the user has positioned the cup portion to cover the ears.

The cup portion 200 may be provided with an area 220 in the vicinity of the upper part 202 where the height of the cup portion 200 is less than the cup portion 200 is in the vicinity of the lower area 203. This reduced height (measured along an ear-to-ear axis during use of the hearing protection device) may be utilized when the cup portion is intended to be inserted under a helmet, hat or other headwear of the user, when a user wears both a hearing protection device as well as a headwear, such as a combat helmet.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering.

Furthermore, the labelling of a first element does not imply the presence of a second element and vice versa.

Although features have been shown and described, it will be understood that they are not intended to limit the claimed invention, and it will be made obvious to those skilled in the art that various changes and modifications may be made without departing from the spirit and scope of the claimed invention. The specification and drawings are, accordingly to be regarded in an illustrative rather than restrictive sense. The claimed invention is intended to cover all alternatives, modifications, and equivalents.

### EXAMPLES

Also, in the following, examples of a hearing protection device, a communication device, and a hearing protection system comprising a hearing protection device and a communication device are disclosed. Any of the features disclosed in the following examples may be implemented in the hearing protection device of the claims as well as in the above specification.

A hearing protection system comprising a hearing protection device and a communication device is disclosed, wherein the hearing protection device comprises a first connector; a first ear protector comprising a first sound attenuation body, a first primary microphone, and a first receiver, wherein the first primary microphone and the first receiver are electrically connected to a first primary terminal and a first receiver terminal of the first connector, respectively, and wherein the first sound attenuation body is configured to cover an outer ear of a user; and a second ear protector comprising a second sound attenuation body, a second primary microphone, and a second receiver, wherein the second primary microphone and the second receiver are electrically connected to a second primary terminal and a second receiver terminal of the first connector, respectively, and wherein the second sound attenuation body is configured to cover an outer ear of a user, and wherein the communication device comprises an interface including a second connector comprising terminals configured for mating with respective terminals of the first connector of the hearing protection device, the communication device comprising a processor configured to: receive a first primary microphone input signal from the first primary microphone; process the first primary microphone input signal for provision of a first output signal for the first receiver based on the first primary microphone input signal, wherein to process the first primary microphone input signal comprises applying noise reduction to the first primary microphone input signal; and output the first output signal to a first receiver terminal of the second connector.

It is an important advantage of the hearing protection device that a hearing protection device with improved flexibility during use is provided. The present hearing protection device allows for easy switching between an earmuff hearing protection device and an earpiece hearing protection device.

The disclosed hearing protection device and hearing protection system is easy to handle and maintain.

Further, the present disclosure allows for improved and complex hearing protection processing due to processing being performed in a communication device.

It is an advantage of the present disclosure that an effective passive (mechanical) sound attenuation is provided with the ear protectors given an available ear protector volume, while at the same time allowing for improved hear-through processing and communication with external devices.

The hearing protection device comprises a first connector. The first connector comprises a number of terminals, such as at least four terminals. The first connector comprises a first primary terminal and a first receiver terminal. The first connector optionally comprises a second primary terminal and a second receiver terminal. The first connector optionally comprises a secondary terminal.

The hearing protection device comprises a first ear protector. The first ear protector comprises a first sound attenuation body, a first primary microphone, and a first receiver, wherein the first primary microphone and the first receiver are electrically connected to a first primary terminal and a first receiver terminal of the first connector, respectively. The first sound attenuation body is configured to cover an outer ear or at least a part of an outer ear of a user. The first ear protector optionally comprises a first cushion configured to encircle an outer ear of the user. The first ear protector may be an earmuff.

The hearing protection device comprises a second ear protector. The second ear protector comprises a second sound attenuation body, a second primary microphone, and a second receiver, wherein the second primary microphone and the second receiver are electrically connected to a second primary terminal and a second receiver terminal of the first connector, respectively. The second sound attenuation body is configured to cover an outer ear or at least a part of an outer ear of a user. The second ear protector may be an earmuff.

The first primary microphone device and the second primary microphone device are configured to receive sound from the surroundings. The first receiver and the second receiver are configured for outputting audio or sound signals towards the respective ears of the user when the hearing protection device is in use.

In one or more exemplary hearing protection devices, the hearing protection device comprises a secondary microphone device electrically connected to a secondary terminal of the first connector. The secondary microphone device may be configured for picking up own voice of a user of the hearing protection device. For example, the hearing protection device optionally comprises a microphone boom with a distal end. The secondary microphone device may be arranged at the distal end of the microphone boom. The secondary microphone device comprises on or more microphones and is configured to provide a secondary microphone input signal on the secondary terminal of the first connector. The second ear protector optionally comprises a second cushion configured to encircle an outer ear of the user.

The hearing protection device may be a passive hearing protection device. A passive hearing protection device is a hearing protection device with no internal power source. A passive hearing protection device allows for a simple ear protector with increased available volume in ear protectors for dampening of sound.

In one or more exemplary hearing protection devices, the first ear protector comprises a first attachment assembly, wherein the first attachment assembly is configured for attachment to a helmet. The second ear protector optionally comprises a second attachment assembly, wherein the second attachment assembly is configured for attachment to a helmet.

The hearing protection device may comprise a headband. The first ear protector may be connected or connectable to a first end of the headband. The second ear protector may be connected or connectable to a second end of the headband.

A communication device is disclosed. The communication device comprises an interface including a second connector comprising terminals configured for mating with respective terminals of the first connector of the hearing protection device.

The second connector comprises a number of terminals, such as at least four terminals. The second connector comprises a first primary terminal and a first receiver terminal. The second connector optionally comprises a second primary terminal and a second receiver terminal. The second connector optionally comprises a secondary terminal.

The interface of the communication device comprises a hearing protection device interface including the second connector for connection to the hearing protection device.

The interface of the communication device may comprise a user interface including one or more buttons, such as a first button, and optionally a radio interface. The first button of the user interface may be configured as a push-to-talk button. The radio interface is configured for connection to a radio unit or a communication system/intercom system. The radio interface may include a third connector for connecting to and communicating with a radio unit or a central communication system/intercom system via a cable.

The third connector may comprise a power terminal for provision of power to the communication device from a power unit, e.g. a battery, of the radio unit or communication system/intercom system when connected to the third connector. Accordingly, the hearing protection system may be configured for receiving power from a radio unit or intercom system via the third connector. This is highly beneficial, since charging of batteries and/or exchange of batteries is not necessary for the hearing protection system, but moved to the radio unit/intercom system, in turn making handling and operation of the hearing protection easier and more reliable.

The communication device optionally comprises a processor. The processor is wired to respective terminals of the hearing protection device interface, the user interface, and the radio interface. The processor may be configured to receive a first primary microphone input signal from the first primary microphone; process the first primary microphone input signal for provision of a first output signal for the first receiver based on the first primary microphone input signal; and output the first output signal to a first receiver terminal of the second connector. By having signal processing taking place in the communication device, a more complex and sophisticated signal processing can take place while allowing for a reduced-size ear protector and/or improved mechanical noise reduction in the ear protector. Further, the same hearing protection device can be readily used for different/next-generation communication devices.

In the processor of the communication device, to process the first primary microphone input signal may comprise performing hear-through processing of the first primary microphone input signal. In the present context hear-through processing is signal processing which improves or adjusts the properties of the input signal for the benefit of the user. In other words, hear-through processing is the ability for the user to hear the surrounding environment, while being protected by the hearing protection system. The hear-through path is the electrical path from the external microphone (first primary microphone and second primary microphone) through the processor and which is then played in the receiver (first receiver and second receiver).

In the processor of the communication device, to process the first primary microphone input signal may comprise, e.g. as part of performing hear-through processing of the first primary microphone input signal, applying noise reduction, e.g. impulse noise reduction and/or broadband noise reduction, to the first primary microphone input signal. Applying noise reduction to the first primary microphone input signal may comprise applying noise reduction to selected frequency bands, such as one, two, three, or more frequency bands, of the first primary microphone input signal.

In one or more exemplary communication devices, the processor may be configured to receive a second primary microphone input signal from the second primary microphone; process the second primary microphone input signal for provision of a second output signal for the second receiver based on the second primary microphone input signal; and output the second output signal to a second receiver terminal of the second connector.

In the processor of the communication device, to process the second primary microphone input signal may comprise performing hear-through processing of the second primary microphone input signal.

In the processor of the communication device, to process the second primary microphone input signal may comprise, e.g. as part of performing hear-through processing of the second primary microphone input signal, applying noise reduction, e.g. impulse noise reduction and/or broadband noise reduction, to the second primary microphone input signal. Applying noise reduction to the second primary microphone input signal may comprise applying noise reduction to selected frequency bands, such as one, two, three, or more frequency bands, of the second primary microphone input signal.

Accordingly, the processor may comprise a hear-through processing module for hear-through processing of the first primary microphone input signal and/or the second primary microphone input signal. The processor, e.g. as part of hear-through processing module, may comprise a first noise reduction module for performing noise reduction, such as impulse noise reduction and/or broadband noise reduction, on the first primary microphone input signal, e.g. for provision of a first primary output signal forming at least part of the first output signal for the first receiver based on the first primary microphone input signal. The processor, e.g. as part of hear-through processing module, may comprise a second noise reduction module for performing noise reduction, such as impulse noise reduction and/or broadband noise reduction, on the second primary microphone input signal, e.g. for provision of second primary output signal forming at least part of second output signal for the second receiver based on the second primary microphone input signal.

In one or more exemplary communication devices, the processor may be configured to receive a secondary microphone input signal from the secondary microphone device; process the secondary microphone input signal for provision of an output audio signal based on the secondary microphone input signal; and output the output audio signal, e.g. to an audio terminal of the radio interface. Thereby, the communication device is able to pick-up and transmit to a radio unit own voice of a user of the hearing protection device.

To process the secondary microphone input signal may comprise applying noise reduction, e.g. impulse noise reduction and/or broadband noise reduction, to the secondary microphone input signal. Applying noise reduction to the secondary microphone input signal may comprise applying noise reduction to selected frequency bands, such as one, two, three, or more frequency bands, of the secondary microphone input signal.

In one or more exemplary communication devices, the processor may be configured to receive an input audio signal via the radio interface, e.g. via the third connector; and form the first output signal and/or the second output signal based on the input audio signal. The processor may be configured to process the input audio signal for provision of a first secondary output signal forming at least a part of the first output signal based on the input audio signal and/or for provision of a second secondary output signal forming at least a part of the second output signal based on the input audio signal. To process the input audio signal and/or to form the first output signal and/or the second output signal based on the input audio signal may comprise applying noise reduction, e.g. impulse noise reduction and/or broadband noise reduction, to the input audio signal. Applying noise reduction to the input audio signal may comprise applying noise reduction to selected frequency bands, such as one, two, three, or more frequency bands, of the input audio signal.

Accordingly, the processor may comprise a communication module configured for provision of a first secondary output signal forming at least part of the first output signal for the first receiver based on input audio signal from the radio interface. The input audio signal may be used directly as the first secondary output signal or processed, e.g. noise-reduced, to form the first secondary output signal. The communication module is optionally configured for provision of second secondary output signal forming at least part of second output signal for the second receiver based on the input audio signal. The input audio signal may be used directly as the second secondary output signal or processed, e.g. noise-reduced, to form the second secondary output signal. The communication module may be configured to provide an output audio signal for the radio unit/communication system/intercom system based on the secondary microphone input signal. The secondary microphone input signal may be fed directly to the radio interface as output audio signal or optionally processed, e.g. noise-reduced, in a voice processing module of the communication module. The voice processing module may be configured to filter and/or amplify the secondary microphone input signal for provision of the output audio signal. Accordingly, the communication module may comprise a voice processing module configured to process the secondary microphone input signal to form the output audio signal. The voice processing module may comprise a noise reduction module for applying noise-reduction to the secondary microphone input signal. In the communication module, to process the secondary microphone input signal may comprise applying noise reduction to the secondary microphone input signal. Applying noise reduction to the secondary microphone input signal optionally comprises applying impulse noise reduction to the secondary microphone input signal. Applying noise reduction to the secondary microphone input signal may comprise applying broadband noise reduction to the secondary microphone input signal. Applying noise reduction to the secondary microphone input signal may comprise applying noise reduction to selected frequency bands, such as one, two, three, or more frequency bands, of the secondary microphone input signal.

The processor of the communication device may comprise a mixing module configured for provision of the first output signal based on the first primary output signal from the hear-through processing module, and the first secondary output signal from the communication module, e.g. by addition and/or a linear combination of the first primary output signal and the first secondary output signal. Further, the mixing module may be configured for provision of the second output signal based on the second primary output signal from the hear-through processing module, and the second secondary output signal from the communication module, e.g. by addition and/or a linear combination of the second primary output signal and the second secondary output signal.

### ITEMS

Also disclosed are hearing protection devices and hearing protection systems according to any of the following items. Any of the features disclosed in the following Items may be implemented in the hearing protection device of the claims as well as in the above specification.
1. A hearing protection system comprising a hearing protection device and a communication device, wherein the hearing protection device comprises
   a first connector;
   a first ear protector comprising a first sound attenuation body, a first primary microphone, and a first receiver, wherein the first primary microphone and the first receiver are electrically connected to a first primary terminal and a first receiver terminal of the first connector, respectively, and wherein the first sound attenuation body is configured to cover an outer ear of a user; and
   a second ear protector comprising a second sound attenuation body, a second primary microphone, and a second receiver, wherein the second primary microphone and the second receiver are electrically connected to a second primary terminal and a second receiver terminal of the first connector, respectively, and wherein the second sound attenuation body is configured to cover an outer ear of a user, and wherein the communication device comprises an interface including a second connector comprising terminals configured for mating with respective terminals of the first connector of the hearing protection device, the communication device comprising a processor configured to:
      receive a first primary microphone input signal from the first primary microphone;
      process the first primary microphone input signal for provision of a first output signal for the first receiver based on the first primary microphone input signal, wherein to process the first primary microphone input signal comprises applying noise reduction to the first primary microphone input signal; and
      output the first output signal to a first receiver terminal of the second connector.
2. Hearing protection system according to item 1, wherein the hearing protection device comprises a secondary microphone device electrically connected to a secondary terminal of the first connector, the secondary microphone device configured for picking up own voice of a user of the hearing protection device.
3. Hearing protection system according to item 2, wherein the hearing protection device comprises a microphone boom with a distal end, and wherein the secondary microphone device is arranged at the distal end of the microphone boom.
4. Hearing protection system according to any of items 1-3, wherein the hearing protection device is a passive hearing protection device.
5. Hearing protection system according to any of items 1-4, wherein the first ear protector comprises a first cushion configured to encircle an outer ear of the user, and wherein the second ear protector comprises a second cushion configured to encircle an outer ear of the user.
6. Hearing protection system according to any of items 1-5, wherein the first ear protector comprises a first attachment assembly, and wherein the second ear protector comprises a second attachment assembly, the first attachment assembly and the second attachment assembly each being configured for attachment to a helmet.
7. Hearing protection system according to any of items 1-5, the hearing protection device comprising a headband, and wherein the first ear protector and the second ear protector are connected to respective first end and second end of the headband.
8. Hearing protection system according to any of items 1-7, wherein the interface comprises a user interface including one or more buttons; and a radio interface for connection with a radio unit and/or an intercom system.
9. Hearing protection system according to any of items 1-8, wherein to process the first primary microphone input signal comprises performing hear-through processing of the first primary microphone input signal.
10. Hearing protection system according to any of items 1-9, wherein applying noise reduction to the first primary microphone input signal comprises applying impulse noise reduction to the first primary microphone input signal.
11. Hearing protection system according to any of items 1-10, wherein the processor is configured to:
   receive a second primary microphone input signal from the second primary microphone;
   process the second primary microphone input signal for provision of a second output signal for the second receiver based on the second primary microphone input signal; and
   output the second output signal to a second receiver terminal of the second connector.
12. Hearing protection system according to item 11, wherein to process the second primary microphone input signal comprises performing hear-through processing of the second primary microphone input signal.
13. Hearing protection device according to any of items 11-12, wherein to process the second primary microphone input signal comprises applying noise reduction to the second primary microphone input signal.
14. Hearing protection system according to any of items 1-13 as dependent on item 2 and item 8, wherein the processor is configured to:
   receive a secondary microphone input signal from the secondary microphone device;
   process the secondary microphone input signal for provision of an output audio signal based on the secondary microphone input signal; and
   output the output audio signal to an audio terminal of the radio interface.
15. Hearing protection system according to any of items 11-14 as dependent on item 8, wherein the processor is configured to:
   receive an input audio signal via the radio interface; and
   form the first output signal based on the input audio signal.
16. A hearing protection device comprising
   a first connector;
   a first ear protector comprising a first sound attenuation body, a first primary microphone, and a first receiver, wherein the first primary microphone and the first receiver are electrically connected to a first primary terminal and a first receiver terminal of the first connector, respectively, and wherein the first sound attenuation body is configured to cover an outer ear of a user; and
   a second ear protector comprising a second sound attenuation body, a second primary microphone, and a second receiver, wherein the second primary microphone and the second receiver are electrically connected to a second primary terminal and a second receiver terminal of the first connector, respectively, and wherein the second sound attenuation body is configured to cover an outer ear of a user.
17. Hearing protection device according to item 16, wherein the hearing protection device comprises a secondary microphone device electrically connected to a secondary terminal of the first connector, the secondary microphone device configured for picking up own voice of a user of the hearing protection device.
18. Hearing protection device according to item 17, wherein the hearing protection device comprises a microphone boom with a distal end, and wherein the secondary microphone device is arranged at the distal end of the microphone boom.
19. Hearing protection device according to any of items 16-18, wherein the hearing protection device is a passive hearing protection device.
20. Hearing protection device according to any of items 16-19, wherein the first ear protector comprises a first cushion configured to encircle an outer ear of the user, and wherein the second ear protector comprises a second cushion configured to encircle an outer ear of the user.
21. Hearing protection device according to any of item 16-20, wherein the first ear protector comprises a first attachment assembly, and wherein the second ear protector comprises a second attachment assembly, the first attachment assembly and the second attachment assembly each being configured for attachment to a helmet.
22. Hearing protection device according to any of items 16-20, the hearing protection device comprising a headband, and wherein the first ear protector and the second ear protector are connected to respective first end and second end of the headband.
23. Hearing protection system comprising a hearing protection device according to any of items 16-22 and a communication device comprising an interface including a second connector comprising terminals configured for mating with respective terminals of the first connector of the hearing protection device.
24. Hearing protection system according to item 23, the interface comprising a user interface including one or more buttons; and a radio interface for connection with a radio unit and/or an intercom system.
25. Hearing protection system according to any of items 23-24, wherein the communication device comprises a processor configured to:
   receive a first primary microphone input signal from the first primary microphone;
   process the first primary microphone input signal for provision of a first output signal for the first receiver based on the first primary microphone input signal; and
   output the first output signal to a first receiver terminal of the second connector.
Item 26. Hearing protection system according to item 25, wherein to process the first primary microphone input signal comprises performing hear-through processing of the first primary microphone input signal.
Item 27. Hearing protection system according to any of items 25-26, wherein to process the first primary microphone input signal comprises applying noise reduction to the first primary microphone input signal.
Item 28. Hearing protection system according to item 27, wherein applying noise reduction to the first primary microphone input signal comprises applying impulse noise reduction to the first primary microphone input signal.
Item 29. Hearing protection system according to any of items 27-28, wherein applying noise reduction to the first primary microphone input signal comprises applying broadband noise reduction to the first primary microphone input signal.
Item 30. Hearing protection system according to any of items 27-29, wherein applying noise reduction to the first primary microphone input signal comprises applying noise reduction to selected frequency bands, such as one, two, three, or more frequency bands, of the first primary microphone input signal.
Item 31. Hearing protection system according to any of items 25-30, wherein the processor is configured to:
   receive a second primary microphone input signal from the second primary microphone;
   process the second primary microphone input signal for provision of a second output signal for the second receiver based on the second primary microphone input signal; and
   output the second output signal to a second receiver terminal of the second connector.
Item 32. Hearing protection system according to item 31, wherein to process the second primary microphone input signal comprises performing hear-through processing of the second primary microphone input signal.
Item 33. Hearing protection system according to any of items 31-32, wherein to process the second primary microphone input signal comprises applying noise reduction to the second primary microphone input signal.
Item 34. Hearing protection system according to item 33, wherein applying noise reduction to the second primary microphone input signal comprises applying impulse noise reduction to the second primary microphone input signal.
Item 35. Hearing protection system according to any of items 33-34, wherein applying noise reduction to the second primary microphone input signal comprises applying broadband noise reduction to the second primary microphone input signal.
Item 36. Hearing protection system according to any of items 33-35, wherein applying noise reduction to the second primary microphone input signal comprises applying noise reduction to selected frequency bands, such as one, two, three, or more frequency bands, of the second primary microphone input signal.
Item 37. Hearing protection system according to any of items 15-36 as dependent on item 17 and item 25, wherein the processor is configured to:
   receive a secondary microphone input signal from the secondary microphone device;
   process the secondary microphone input signal for provision of an output audio signal based on the secondary microphone input signal; and
   output the output audio signal to an audio terminal of the radio interface.
Item 38. Hearing protection system according to item 37, wherein to process the secondary microphone input signal comprises applying noise reduction to the secondary microphone input signal.
Item 39. Hearing protection system according to item 38, wherein applying noise reduction to the secondary microphone input signal comprises applying impulse noise reduction to the secondary microphone input signal.
Item 40. Hearing protection system according to any of items 38-39, wherein applying noise reduction to the secondary microphone input signal comprises applying broadband noise reduction to the secondary microphone input signal.
Item 41. Hearing protection system according to any of items 38-40, wherein applying noise reduction to the secondary microphone input signal comprises applying noise reduction to selected frequency bands, such as one, two, three, or more frequency bands, of the secondary microphone input signal.
Item 42. Hearing protection system according to any of items 31-41 as dependent on item 16, wherein the processor is configured to:
   receive an input audio signal via the radio interface; and
   form the first output signal based on the input audio signal.
Item 43. Hearing protection system according to item 42, wherein to form the first output signal based on the input audio signal comprises applying noise reduction to the input audio signal.
Item 44. Hearing protection system according to item 43, wherein applying noise reduction to the input audio signal comprises applying impulse noise reduction to the input audio signal.
Item 45. Hearing protection system according to any of items 42-43, wherein applying noise reduction to the input audio signal comprises applying broadband noise reduction to the input audio signal.
Item 46. Hearing protection system according to any of items 42-45, wherein applying noise reduction to the input audio signal comprises applying noise reduction to selected frequency bands, such as one, two, three, or more frequency bands, of the input audio signal.
Item 47. Hearing protection system according to any of items 23-46 as dependent on item 24, wherein the radio interface comprises a third connector comprising a power terminal for provision of power to the communication device from a radio unit connected to the third connector.

### LIST OF REFERENCES

- 2: hearing protection system
- 4: hearing protection device
- 6: communication device
- 8: first connector
- 10: first ear protector
- 12: second ear protector
- 14A: first sound attenuation body
- 14B: second sound attenuation body
- 16: first primary microphone
- 16A: first primary microphone input signal
- 18: first receiver
- 18A: first output signal
- 18B: first primary output signal
- 18C: first secondary output signal
- 20: second primary microphone
- 20A: second primary microphone input signal
- 22: second receiver
- 22A: second output signal
- 22B: second primary output signal
- 22C: second secondary output signal
- 24: secondary microphone device
- 24A: secondary microphone input signal
- 26: microphone boom
- 28: distal end
- 30: first cushion
- 32: second cushion
- 34: headband
- 36: first end of headband
- 38: second end of headband
- 50: processor
- 52: hearing protection device interface
- 54: user interface
- 56: first button
- 58: radio interface
- 60: third connector
- 70: radio unit
- 72: cable
- 74: second connector
- 80: first primary terminal of first connector
- 80A: first primary terminal of second connector
- 82: first receiver terminal of first connector
- 82A: first receiver terminal of second connector
- 84: second primary terminal of first connector
- 84A: second primary terminal of second connector
- 86: second receiver terminal of first connector
- 86A: second receiver terminal of second connector
- 88: secondary terminal of first connector
- 88A: secondary terminal of second connector
- 90: reference (ground) terminal of first connector
- 90A: reference (ground) terminal of second connector
- 100: first primary input of processor
- 102: first receiver output of processor
- 104: second primary input of processor
- 106: second receiver output of processor
- 108: secondary input of processor
- 110: hear-through processing module
- 110A: first noise reduction module
- 110B: second noise reduction module
- 112: communication module
- 114: input audio signal
- 116: output audio signal
- 118: voice processing module
- 120: mixing module
- 122: controller
- 124: control signal
- 126: control signal
- 128: control signal
- 130: user input signal from user interface
- 200: Cup portion
- 201: Outer surface
- 202: Upper part
- 203: Lower part
- 204: Rearward facing part
- 205: Forward facing part
- 206: Connecting part
- 207: Microphone port
- 208: First sound reflector
- 209: Second sound reflector
- 210: Third sound reflector
- 211: Reflector structure
- 212: Inner part
- 213: Shell
- 214: Annular peripheral part
- 215: Inner volume
- 216: Inner surface
- 217: Side wall
- 218: Bottom wall
- 219: Microphone opening
- 220: Cable opening

## Claims

1. A hearing protection device comprising a first ear protector, the first ear protector comprising at least a first sound attenuation body, a first receiver, and a first primary microphone, the first sound attenuation body comprising a cup portion providing a noise dampening space, the cup portion having a peripheral part comprising a forward facing part, a rearward facing part, an upper part and a lower part, the cup portion further comprising:
- an outer surface facing the ambient space;
- an inner cup portion accommodating the first receiver;
- a primary sound reception area positioned in a central area of the outer surface of the cup portion, where the central area is in an area which is between 20 and 80 % of the length between the forward facing part and the rearward facing part; and
- at least a first sound reflector configured to reflect and direct sound waves towards the primary sound reception area, where the first sound reflector is positioned in an area on the outer surface between the sound reception area and the peripheral part of the cup portion.

2. A hearing protection device according to claim 1, wherein the hearing protection device comprises a second ear protector, the second ear protector comprising a second attenuation body, where the second ear protector is configured to be connected to the first attenuation body via a connecting element.

3. A hearing protection device according to any of the preceding claims, wherein the first sound reflector is positioned between the rearward facing part of the peripheral part and the primary sound reception area.

4. A hearing protection device according to any of the preceding claims, wherein the cup portion further comprises a second sound reflector where the second sound reflector is positioned between the upper part of the peripheral part and the primary sound reception area.

5. A hearing protection device according to any of the preceding claims, wherein the outer cup portion further comprises a third sound reflector where the third sound reflector is positioned between a lower part of the peripheral part and the primary sound reception area.

6. A hearing protection device according to any of the preceding claims, wherein sound reflector further comprises a fourth sound reflector where the fourth sound reflector is positioned between the forward facing part of the peripheral part and the primary sound reception area.

7. A hearing protection device according to any of the preceding claims, wherein the outer cup portion comprises a channel extending from the sound reception area in a direction towards the front facing part of the peripheral edge, where the at least first sound reflector optionally abuts the channel.

8. A hearing protection device according to any of the preceding claims, wherein the sound reception area comprises a microphone opening for directing incoming sound to the first primary microphone.

9. A hearing protection device according to any of the preceding claims, wherein the sound reception area is offset from the outer surface of the cup portion in a radial direction

10. A hearing protection device according to any of the preceding claims, wherein the sound reception area comprises an opening in the outer surface of the cup portion.

11. A hearing protection device according to any of the preceding claims, wherein the sound reception area comprises a canal extending from the outer surface of the cup portion and extending inwards towards the inner portion of the cup portion.

12. A hearing protection device according to any of the preceding claims, wherein the sound receiving area is configured to be positioned substantially centered relative to the ear canal of the user, when the hearing protection device is in use.

13. A hearing protection device according to any of the preceding claims, wherein the first ear protector is an over-the-ear configuration.

14. A hearing protection device according to any of the preceding claims, wherein the peripheral edge of the cup portion is provided with a cushion providing a seal between the side of the head and the cup portion.

15. A hearing protection device according to any of the preceding claims, wherein the hearing protection device comprises a secondary microphone device wired to a secondary terminal of the first connector, the secondary microphone device configured for picking up own voice of a user of the hearing protection device.
